(19) **Europäisches Patentamt**
**European Patent Office**
**Office européen des brevets**

(11) **EP 2 789 335 B1**

(12) **EUROPEAN PATENT SPECIFICATION**

(45) Date of publication and mention
of the grant of the patent:
**15.02.2017 Bulletin 2017/07**

(21) Application number: **12854655.3**

(22) Date of filing: **06.12.2012**

(51) Int Cl.:
*A61K 31/196* (2006.01)   *A61K 9/70* (2006.01)
*A61K 47/02* (2006.01)   *A61K 47/10* (2017.01)
*A61K 47/34* (2017.01)   *A61P 29/00* (2006.01)

(86) International application number:
**PCT/JP2012/081657**

(87) International publication number:
**WO 2013/084995 (13.06.2013 Gazette 2013/24)**

(54) **ADHESIVE PATCH**

KLEBEPFLASTER

TIMBRE ADHÉSIF

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **07.12.2011   JP 2011267470**

(43) Date of publication of application:
**15.10.2014 Bulletin 2014/42**

(73) Proprietor: **Hisamitsu Pharmaceutical Co., Inc.
Tosu-shi, Saga 841-0017 (JP)**

(72) Inventors:
• **KIJIMA Masaru
Tosu-shi
Saga 841-0017 (JP)**

• **KOSE Yasuhisa
Tosu-shi
Saga 841-0017 (JP)**
• **YOSHINAGA Takaaki
Tosu-shi
Saga 841-0017 (JP)**

(74) Representative: **Grünecker Patent- und
Rechtsanwälte
PartG mbB
Leopoldstraße 4
80802 München (DE)**

(56) References cited:
**JP-A- H0 656 660      JP-A- H03 109 327
JP-A- H08 319 213      JP-A- H10 182 450
JP-A- 2001 064 161**

**Description**

[Technical Field]

The present invention relates to a patch.

[Background Art]

Diclofenac is a nonsteroidal analgesic anti-inflammatory drug, and is listed in Pharmacopoeias of countries around the world including The Japanese Pharmacopoeia. Pharmaceutical preparations containing diclofenac sodium, which is a salt of diclofenac, .are not limited to oral preparations such as tablets and capsules. Recently, topical agents containing diclofenac sodium, such as gels, ointments, and patches, have been provided, and widely used for.treatment of localized diseases such as osteoarthritis and shoulder periarthritis.

Attempts to achieve stable release of diclofenac from such topical agents have been made conventionally, and, for example, Japanese Unexamined Patent Application Publication No. Sho 63-091318 (PTL 1) describes a patch containing diclofenac sodium and 1,3-butylene glycol. Moreover, Japanese Unexamined Patent Application Publication No. Hei 6-56660 (PTL 2) describes a patch containing diclofenac sodium, water, a polyvalent alcohol fatty acid ester, and 1, 3-butylene glycol as a solubilizer. However, these patches still have a problem of insufficiency in skin permeability of diclofenac.

In addition, as a patch for improving the skin permeability of diclofenac, Japanese Unexamined Patent Application Publication No. Hei 10-182450 (PTL 3) describes a patch containing diclofenac, an alcohol such as propylene glycol or glycerin, and a carboxylic acid ester such as a surfactant. Polyethylene glycol fatty acid esters and the like are listed as examples of the surfactant. Moreover, Japanese Unexamined Patent Application Publication No. 2002-193793 (PTL 4) describes a patch containing diclofenac sodium, glycerin, and glycol. In addition, propylene glycol, 1,3-butylene glycol, and the like are listed as examples of the glycol.

Moreover, Japanese Unexamined Patent Application Publication No. Sho 61-060608 (PTL 5) states that the skin permeability of diclofenac is improved when a poultice containing diclofenac sodium and water contains analkylene glycol such as propanediol (propylene glycol) orbutanediol (butylene glycol) and a base agent such as gelatin. Japanese Unexamined Patent Application Publication No. Hei 3-109321 (PTL 6) states that the skin permeability of diclofenac is improved when a pharmaceutical gel preparation contains diclofenac and a nonionic surfactant having an HLB value of 14.5 or lower. Propylene glycol is used as a co-solvent in the pharmaceutical gel preparation.

However, use of propylene glycol for each of the above-described patches poses a problem that the patch causes skin symptoms of redness, erythema, rash, itching, and pain, and gives a pain during peeling off, and a problem that the adhesiveness to the skin is insufficient. In addition, use of glycerin instead of propylene glycol for the purpose of reducing the irritation to the skin causes a problem of decrease in skin permeability of diclofenac.

[Citation List]

[Patent Literature]

[PTL 1] Japanese Unexamined Patent Application Publication No. Sho 63-091318
[PTL 2] Japanese Unexamined Patent Application Publication No. Hei 6-56660
[PTL 3] Japanese Unexamined Patent Application Publication No. Hei 10-182450
[PTL 4] Japanese Unexamined Patent Application Publication No. 2002-193793
[PTL 5] Japanese Unexamined Patent Application Publication No. Sho 61-060608
[PTL 6] Japanese Unexamined Patent Application Publication No. Hei 3-109321

[Summary of Invention]

[Technical Problem]

The present invention has been made in view of the above-described problems of the conventional technologies, and an object of the present invention is to provide a patch which gives reduced pain during peeling off and less irritation to the skin, and which has an excellent skin permeability of diclofenac and an excellent adhesiveness.

[Solution to Problem]

The present inventors have conducted earnest study to achieve the above-described object. As a result, the present inventors have found that the skin permeability of diclofenac is improved without inclusion of propylene glycol or the like, which causes skin irritation, by a patch comprising a support layer and an adhesive agent layer, wherein the adhesive agent layer contains a combination of diclofenac or a pharmaceutically acceptable salt thereof, glycerin, butylene glycol, and poly(ethylene glycol) monooleate, and a mass ratio between the butylene glycol and the poly(ethylene glycol) monooleate is set within a specific range. Moreover, the present inventors have found that the patch has an excellent adhesiveness, and gives s sufficiently reduced pain during peeling off.

In addition, acceleration of decomposition of diclofenac sodium, which is a salt of diclofenac, precipitation of diclofenac sodium, and the like occurring in a case where glycerin and diclofenac sodium are contained in an adhesive agent layer of a patch have been reported so far in PTLs 1 and 5 listed above, and the like. In contrast, the present inventors have astonishingly found that by making the patch have the above-described specific constitution, the skin permeability of diclofenac can be improved even in a case where glycerin is used with diclofenac or a salt thereof, so that the patch which contains glycerin and which gives less skin irritation can be obtained.

Moreover, the present inventors have found that the effect of improving the skin permeability of diclofenac achieved by the patch is remarkably better than those achieved in cases where butylene glycol or poly(ethylene glycol) monooleate mentioned above is used alone, and that this effect is a synergistic effect achieved by combining the diclofenac, the glycerin, the butylene glycol, and the poly (ethylene glycol) monooleate each other, with the ratio between the butylene glycol and the poly(ethylene glycol) monooleate being a specific ratio. These findings have led to the completion of the present invention.

Specifically, a patch of the present invention comprises a support layer and an adhesive agent layer, wherein the adhesive agent layer contains diclofenac or a pharmaceutically acceptable salt thereof, glycerin, butylene glycol, and poly(ethylene glycol) monooleate, and

a mass ratio between the butylene glycol and the poly(ethylene glycol) monooleate (mass of butylene glycol:mass of poly(ethylene glycol) monooleate) is 1:1 to 4:1.

In the patch of the present invention, a content of the butylene glycol is preferably 4 to 18% by mass relative to a total mass of the adhesive agent layer, and a content of the poly(ethylene glycol) monooleate is preferably 2.5 to 7.5% by mass relative to the total mass of the adhesive agent layer.

Moreover, in the patch of the present invention, the adhesive agent layer preferably further contains sodium sulfite. In addition, an average number of moles of oxyethylene groups added in the poly(ethylene glycol) monooleate is preferably 2 to 10. Furthermore, the butylene glycol is preferably 1,3-butylene glycol.

[Advantageous Effects of Invention]

According to the present invention, it is possible to provide a patch which gives reduced pain during peeling off and less irritation to the skin, and which has an excellent skin permeability of diclofenac and an excellent adhesiveness.

[Description of Embodiments]

Hereinafter, the present invention will be described in detail based on preferred embodiments thereof.

A patch of the present invention comprises a support layer and an adhesive agent layer formed on a surface or surfaces (generally on one of the surfaces) of the support layer, and the adhesive agent layer contains diclofenac or a pharmaceutically acceptable salt thereof, glycerin, butylene glycol, and poly(ethylene glycol) monooleate. Examples of the patch include poultices in which the adhesive agent layer contains water, and plasters in which the adhesive agent layer does not contain water. The patch of the present invention is preferably a poultice from the viewpoints that a poultice tends to have an excellent compatibility with glycerin and the like, and that a rapid-cooling effect on acute diseases such as bruises tends to be expected because of action of the.contained water.

Diclofenac according to the present invention is a nonsteroidal drug having analgesic and anti-inflammatory effects. A form of the diclofenac may be a free form or a form of a pharmaceutically acceptable salt thereof. The free form may be achieved by desalination of a salt of diclofenac during production and/or in a pharmaceutical preparation after production. The form of the diclofenac may be one of such forms or a mixture of two or more thereof. Of these forms, the form of the diclofenac according to the present invention is preferably a pharmaceutically acceptable salt of diclofenac from the viewpoint that the stability of the drug tends to be improved, so that irritation to the skin and decreases in physical properties (strength, elasticity, durability, tackiness, and when the patch is a poultice, water retaining ability) of the adhesive agent layer caused by an acid can be suppressed. Examples of the pharmaceutically acceptable salt of diclofenac include, sodium salt, epolamine salt (1-(2-hydroxyethyl)pyrrolidine salt), and ammonium salt. Of these salts, sodium salt is preferable.

In the patch of the present invention, a total content of the diclofenac and the pharmaceutically acceptable salts thereof can be adjusted, as appropriate, depending on the purpose of therapy, and is generally 0.3 to 15% by mass relative to a total mass of the adhesive agent layer. The total content is preferably 0.5 to 5% by mass from the viewpoints that skin permeability of diclofenac tends to be excellent and that crystal precipitation of diclofenac in the adhesive agent layer tends to be more suppressed.

Since the adhesive agent layer of the patch of the present invention contains glycerin, irritation (redness, erythema, rash, itching, pain, and the like) of the patch to the skin is reduced. A content of the glycerin is preferably 5 to 35% by mass relative to the total mass of the adhesive agent layer. If the content of the glycerin is less than the lower limit, adhesiveness to the skin and sense of use tend to be impaired. Meanwhile, if the content of the glycerin exceeds the upper limit, the shape retainability of the adhesive agent layer tends to decrease, so that a component of the adhesive agent layer seeps into the support layer, the skin permeability of diclofenac tends to decrease, and the storage stability of the drugs including diclofenac tends to decrease, so that the contents of the drugs in the adhesive agent layer decrease. Note that acceleration of decomposition of diclofenac sodium, precipitation of diclofenac sodium, and the like occurring when glycerin and diclofenac sodium, which is a diclofenac salt, are contained in an adhesive agent layer of a patch have been reported so far. However, in the present invention, even when the glycerin is used with the diclofenac and the salt thereof, the skin permeability of diclofenac can be remarkably improved, and the irritation to the skin can be sufficiently reduced.

The butylene glycol according to the present invention includes structural isomers thereof, which differs in arrangement of the hydroxy groups. Of these isomers, the butylene glycol is preferably 1,3-butylene glycol from the viewpoint that butylene glycol generally used for pharmaceutical preparations is 1,3-butylene glycol. Note that 1,3-butylene glycol is an almost odorless, colorless, transparent viscous liquid. In the present invention, the skin permeability of diclofenac can be improved by adding a combination of the butylene glycol with the other components according to the present invention to the adhesive agent layer, and the adhesiveness can also be improved.

A content of the butylene glycol is preferably 1 to 25% by mass relative to the total mass of the adhesive agent layer, and more preferably 4 to 18% by mass. If the content of the butylene glycol is less than the lower limit, the skin permeability of diclofenac tends to decrease. Meanwhile, if the content of the butylenes glycol exceeds the upper limit, the shape retainability of the adhesive agent layer tends to decrease, although the skin permeability of diclofenac is improved.

The poly(ethylene glycol) monooleate according to the present invention is a monooleic acid ester of polyethylene glycol. In the present invention, the adhesive agent layer contains the poly(ethylene glycol) monooleate in combination with the other components according to the present invention. Hence, it is not necessary to add a component having a dissolving effect such as diisopropyl adipate. In the poly(ethylene glycol) monooleate, an average number of moles of oxyethylene groups added in the polyethylene glycol chain is preferably 2 to 20. If the average number of moles of oxyethylene groups added is less than the lower limit, the lipophilicity of the poly(ethylene glycol) monooleate tends to be high. Meanwhile, if the average number exceeds the upper limit, the hydrophilicity tends to be high. Especially, the average number of moles of oxyethylene groups added is more preferably 2 to 10 from the viewpoint that the balance between the lipophilicity and the hydrophilicity is better. The average number of moles of oxyethylene groups added is further preferably 6 or 10, and is particularly preferably 6 from the viewpoint that such poly(ethylene glycol) monooleate is easy to obtain. Note that the average number of moles of oxyethylene groups added can be determined by NMR measurement.

A content of the poly(ethylene glycol) monooleate is preferably 0.5 to 10% by mass and more preferably 2.5 to 7.5% by mass relative to the total mass of the adhesive agent layer. If the content of the poly(ethylene glycol) monooleate is less than the lower limit, the skin permeability of diclofenac tends to decrease. Meanwhile, if the content of the poly(ethylene glycol) monooleate exceeds the upper limit, the irritation to the skin tends to increase, and a bleeding phenomenon tends to occur in which the poly(ethylene glycol) monooleate separates and seeps to a surface of the adhesive agent layer, so that the adhesiveness to the skin decreases.

In addition, in the adhesive agent layer according to the present invention, a mass ratio between the butylene glycol and the poly(ethylene glycol) monooleate (mass of butylene glycol:mass of poly(ethylene glycol) monooleate) has to be 1:1 to 4:1, and is particularly preferably 1.25:1 to 4:1. In the present invention, a cumulative skin permeation amount of diclofenac in 12 hours from the start of a test is preferably 8 $\mu g/cm^2$/12 hr or more in a skin permeation test using the skin of hairless mouse. If the ratio of the mass of the butylene glycol to the mass of the poly (ethylene glycol) monooleate is less than the lower limit, the cumulative skin permeation amount decreases, i.e., the skin permeability of diclofenac decreases. Meanwhile, if the ratio exceeds the upper limit, the shape retainability of the adhesive agent layer decreases, although the cumulative skin permeation amount increases, i.e., the skin permeability of diclofenac is improved.

Moreover, in the adhesive agent layer according to the present invention, a total content of the butylene glycol and the poly(ethylene glycol) monooleate can be adjusted, as appropriate, according to the amount of the diclofenac, and is preferably 1 . 5 to 35% by mass, and more preferably 5 to 25% by mass, relative to the total mass of the adhesive agent layer. If the total content is less than the lower limit, the skin permeability of diclofenac tends to decrease. Meanwhile, if the total content exceeds the upper limit, the shape retainability of the adhesive agent layer tends to decrease, so that a component of the adhesive agent layer seeps into the support layer, the skin permeability of diclofenac tends to decrease, the irritation to the skin tends to increase, and a bleeding phenomenon tends to occur in which the butylene glycol separates and seeps to a surface of the adhesive agent layer, so that the adhesiveness to the skin decreases.

In the patch of the present invention, the adhesive agent layer preferably further contains sodium sulfite. The present inventors have found that when the adhesive agent layer of the patch contains the poly (ethylene glycol) monooleate, the color of the adhesive agent layer gradually changes to brown with lapse of time. In this respect, the present inventors have conducted further study, and consequently found that when the adhesive agent layer further contains sodium sulfite, the change in color of the adhesive agent layer can be sufficiently suppressed.

Examples of the sodium sulfite include sodium sulfite (crystalline) having seven molecules of water of crystallization, anhydrous dried sodium sulfite, and the like. Of these, dried sodium sulfite is preferable. When the adhesive agent layer contains such a sodium sulfite, the content of the sodium sulfite is preferably 0.01 to 1% by mass, and more preferably 0.01 to 0.25% by mass, relative to the total mass of the adhesive agent layer. In addition, when the adhesive agent layer does not contain any one of sodium pyrosulfite and sodium hydrogen sulfite described later, the content of the sodium sulfite is further preferably 0.02 to 0.25% by mass, and particularly preferably 0.03 to 0.2% by mass. If the content of the sodium sulfite is less than the lower limit, it tends to be difficult to sufficiently suppress the change in color of the adhesive agent layer. Meanwhile, if the content of the sodium sulfite exceeds the upper limit, unpleasant putrid odor tends to increase, and the shape retainability of the adhesive agent layer tends to decrease, so that a component of the adhesive agent layer seeps into the support layer, although the effect of suppressing the change in color is improved according to the content.

In addition, in the patch of the present invention, it is also preferable that the adhesive agent layer contain sodium pyrosulfite and/or sodium hydrogen sulfite, in addition to sodium sulfite described above. When the adhesive agent layer contains the sodium pyrosulfite and/or sodium hydrogen sulfite, the content of the sodium sulfite is preferably 0.01 to 0.4% by mass, and more preferably 0.01 to 0.04% by mass, relative to the total mass of the adhesive agent layer. In addition, a total content of the sodium pyrosulfite and the sodium hydrogen sulfite is preferably 0.01 to 0.4% by mass, relative to the total mass of the adhesive agent layer. If the contents of the sodium sulfite, the sodium pyrosulfite, and the sodium hydrogen sulfite are less than the lower limits, it tends to be difficult to sufficiently suppress the change in color of the adhesive agent layer. Meanwhile, if the contents exceed the upper limits, unpleasant putrid odor tends to be strong, although the effect of suppressing the change in color is improved according to the contents.

When the patch of the present invention is a poultice, the adhesive agent layer (drug-containing layer) according to the present invention preferably further contains water and a water-soluble polymer, and may contain a cross-linking agent and the like, if necessary. The water is preferably water purified by ion exchange, distillation, filtration, or the like, and, for example, "purified water" listed in The Japanese Pharmacopoeia (The Japanese Pharmacopoeia Fifteenth Edition) can' be used preferably. A content of the water is preferably 15 to 75% by mass relative to the total mass of the adhesive agent layer (drug-containing layer). If the content of water is out of the range, it tends to be difficult to maintain preferred gel properties (strength, elasticity, durability, tackiness, water retaining ability, and the like) of the adhesive agent layer (drug-containing layer).

Examples of the water-soluble polymer include partially neutralized polyacrylic acid, completely neutralized, polyacrylic acid, polyacrylic acid, carboxyvinyl polymer, carboxymethyl cellulose, carboxymethyl cellulose sodium salt, methyl cellulose, ethyl cellulose, hydroxyethyl cellulose, hydroxypropyl cellulose, gelatin, casein, pullulan, agar, dextran, dextrin, sodium alginate, soluble starch, carboxylated starch, polyvinyl alcohol, polyethylene oxide, polyacrylamide, polyvinylpyrrolidone, polyvinyl ether-maleic anhydride copolymer, methoxyethylene-maleic anhydride copolymer, isobutylene-maleic anhydride copolymer, polyethylenimine, and the like. One of these water-soluble polymers may be used alone, or two or more thereof may be used in combination. The water-soluble polymer is preferably completely neutralized polyacrylic acid, hydroxypropyl cellulose, carboxymethyl cellulose sodium salt, or gelatin, from the viewpoints that gel properties (strength, elasticity, durability, tackiness, water retaining ability, and the like) preferred for a poultice tend to be obtained, and the adhesiveness to the skin tends to be more improved. A total content of such water-soluble polymers is preferably 5 to 20% by mass relative to the total mass of the adhesive agent layer (drug-containing layer) from the viewpoint that further preferred gel properties tend to be obtained.

The cross-linking agent has an effect of cross-linking the water-soluble polymer to convert the adhesive agent layer (drug-containing layer) to a gel for shape retention. The cross-linking agent is not particularly limited, and examples thereof include potassium aluminum sulfate, calcium chloride, magnesium chloride, aluminum hydroxide, dihydroxyaluminum aminoacetate, and magnesium aluminometasilicate. One of these cross-linking agents may be used alone, or two or more thereof may be used in combination. When the cross-linking agents are contained in the adhesive agent layer (drug-containing layer), a total content of the cross-linking agents is preferably 0.1 to 5% by mass relative to the total mass of the adhesive agent layer (drug-containing layer) from the viewpoint that preferred gel properties (strength, elasticity, durability, tackiness, water retaining ability, and the like) tend to be obtained.

Meanwhile, when the patch of the present invention is a plaster, the adhesive agent layer according to the present invention preferably further contains an adhesive agent, and, if necessary, may contain a tackifier, a softener, and the like. Examples of the adhesive agent include acrylic adhesive agents and styrene block copolymer-based adhesive agents from the viewpoints of excellent tackiness and excellent drug release property. One of these adhesive agents may be used alone, or two or more thereof may be used in combination.

The acrylic adhesive agent may be an adhesive agent obtained by polymerization or copolymerization of at least one of (meth) acrylic monomers such as (meth)acrylic acid, 2-ethylhexyl (meth)acrylate, methyl (meth)acrylate, butyl (meth)acrylate, hydroxyethyl (meth)acrylate, and the like. The acrylic adhesive agent is preferably 2-ethylhexyl acrylate·vinyl acetate copolymer, 2-ethylhexyl acrylate·vinyl acetate·acrylic acid copolymer, 2-ethylhexyl acrylate·vinyl acetate·hydroxyethyl acrylate copolymer, 2-ethylhexyl acrylate·vinyl acetate·hydroxyethyl acrylate·acrylic acid copolymer, 2-ethylhexyl acrylate·2-ethylhexyl methacrylate·dodecyl methacrylate copolymer, or the like, and is more preferably 2-ethylhexyl acrylate·vinyl acetate copolymer or 2-ethylhexyl acrylate·vinyl acetate·acrylic acid copolymer.

Examples of the styrene block copolymers include styrene-isoprene-styrene block copolymer (SIS), styrene-butadiene-styrene block copolymer (SBS), styrene-ethylene-butylene-styrene block copolymer (SEBS), styrene-ethylene-propylene-styrene block copolymer (SEPS), and the like. Of these styrene block copolymers, styrene-isoprene-styrene block copolymer (SIS) is preferable.

Examples of the tackifier include alicyclic saturated hydrocarbon resins, rosin and rosin derivatives (for example, rosin glycerin ester, hydrogenated rosin, hydrogenated rosin glycerin ester, rosin pentaerythritol ester, and the like), terpene resins, petroleum resins, maleic acid resin, and the like. Of these tackifiers, alicyclic saturated hydrocarbon resins and hydrogenated rosin glycerin ester are preferable. One of these tackifiers may be used alone, or two or more thereof may be used in combination.

Examples of the softener include petroleum-based oils (for example, paraffinic process oils, naphthenic process oils, aromatic process oils, and the like), squalane, squalene, vegetable-based oils (for example, almond oil, olive oil, camellia oil, castor oil, tall oil, peanut oil, and the like), saturated or unsaturated fatty acids, silicon oil, dibasic acid esters (for example, dibutyl phthalate, dioctylphthalate, and the like),liquid rubbers (for example, polybutene, liquid isoprene rubber, and the like), liquid fatty acid.esters (isopropylmyristate, hexyl laurate, diethyl sebacate, isopropyl sebacate, and the like), diethylene glycol, glycol salicylate, dipropylene glycol, triacetin, triethyl citrate, crotamiton, and the like. One of these softeners may be used alone, or two or more thereof may be used in combination. The softener is preferably liquid paraffin and/or isopropyl myristate from the viewpoint that the adhesiveness to the skin tends to be further improved.

In addition, the adhesive agent layer according to the present invention may further contain polyvalent alcohols other than the glycerin and the butylene glycol, a pH-adjusting agent, an antiseptic, an antioxidant, and other additives, unless an effect of.the present invention is impaired.

The polyvalent alcohols may be alcohols other than the glycerin and the butylene glycol, and examples thereof include propylene glycol, polyethylene glycol, D-sorbitol, and the like. One of these polyvalent alcohols may be used alone, or two or more thereof may be used in combination. When such polyvalent alcohols are contained in the adhesive agent layer, a total content of the polyvalent alcohols is preferably 60% by mass or less, and more preferably 15 to 60% by mass, relative to the total mass of the adhesive agent layer, from the viewpoint that the adhesive agent layer tends to be able to retain a preferred water retention ability, a preferred drug solubility, and a preferred tackiness in a well-balanced manner. In addition, of these polyvalent alcohols, when propylene glycol is used, the content is particularly preferably 10% by mass or less relative to the total mass of the adhesive agent layer from the viewpoint that the irritation to the skin tends to increase, although the solubility and skin permeability of diclofenac are improved. Note that, of these polyvalent alcohols, it is not preferable to add sorbitol, from the viewpoint that when sorbitol is contained in the adhesive agent layer, the stability of the patch may be lowered in some cases.

The pH-adjusting agent has an effect of adjusting the skin permeation rate of the drug and the irritation to the skin of the patch, and, when the patch of the present invention is a poultice, an effect of adjusting the cross-linking speed. Examples of the pH-adjusting agent include organic acids such as acetic acid, lactic acid, oxalic acid, citric acid, tartaric acid, and edetic acid; and inorganic acids such as hydrochloric acid, sulfuric acid, nitric acid, and phosphoric acid; as well as pharmaceutically acceptable salts of the organic acids and the inorganic acids. One of these pH-adjusting agents may be used alone, or two or more thereof may be used in combination. When such pH-adjusting agents are contained in the adhesive agent layer, a total content of the pH-adjusting agents can be adjusted, as appropriate, depending on a target pH. The total content of the pH-adjusting agents is preferably 0.05 to 2% by mass relative to the total mass of the adhesive agent layer, from the viewpoint that preferred physical properties (strength, elasticity, durability, tackiness, and when the patch is a poultice, water retaining ability) of the adhesive agent layer tend to be obtained. Note that when the patch of the present invention is a poultice, the pH of the adhesive agent layer is preferably 5.5 to 8.0 from the viewpoints that the skin permeability of the drug tends to-be improved, the storage stability of the pharmaceutical preparation tends to be improved, and further a preferred cross-linking speed tends to be achieved. Note that the pH herein is a value obtained by suspending 5 parts by mass of the adhesive agent layer in 95 parts by mass of purified water, and measuring the suspension with a combination glass electrode.

Examples of the antiseptic include methyl paraoxybenzoate, ethyl paraoxybenzoate, propyl paraoxybenzoate, butyl paraoxybenzoate, 1,2-pentanediol, benzoic acid, salts thereof, salicylicacid, salts thereof, sorbic acid, salts thereof, dehydroacetic acid, salts thereof, 4-isopropyl-3-methylphenol, 2-isopropyl-5-methylphenol, phenol, hinokitiol, cresol, 2,4,4'-trichloro-2'-hydroxydiphenyl ether, 3,4,4'-trichlorocarbanilide, chlorobutanol, benzalkonium chloride, benzethonium chloride, and the like. One of these antiseptics may be used alone, or two or more' thereof may be used in combination. When such antiseptics are'contained in the adhesive agent layer, a total content of the antiseptics is preferably 0.005 to 1% by mass relative to the total mass of the adhesive agent layer, from the viewpoint that a preferred antiseptic effect tends to be achieved.

Examples of the antioxidant include butylhydroxyanisole, dibutylhydroxytoluene, nordihydroguaiaretic acid, tocopherols, tocopheryl acetate, citric acid, edetic acid, ascorbic acid, propyl gallate, and the like. One of these antioxidants may be used alone, or two or more thereof may be used in combination. When such antioxidants are contained in the adhesive agent layer, a total content of the antioxidants is preferably 0.001 to 5% by mass relative to the total mass of the adhesive agent layer from the viewpoint that a preferred antioxidative effect tends to be achieved.

Examples of other additives mentioned above include inorganic fillers (inorganic bulking agents) such as titanium oxide, aluminum silicate, light anhydrous silicic acid, and kaolin; menthol; a tackiness-improving agent made of an acrylic acid ester copolymer such as aminoalkyl methacrylate copolymer E; and the like. One of these additives may be used alone, or two or more thereof may be used in combination.

The adhesive agent layer according to the present invention is a layer containing the above-described components, and may be a single layer having a single constitution or a multilayer in which multiple layers having different constitutions are stacked on each other. A thickness of the adhesive agent layer is preferably 250 to 1500 $\mu$m from the viewpoint that the skin permeability of diclofenac is better.

Examples of materials of the support layer according to the present invention include polyethylene, polypropylene, ethylene-vinyl acetate copolymer, vinyl chloride, polyurethane, polyesters such as polyethylene terephthalate and polybutylene terephthalate; polyamides such as nylon; celluloses and derivatives thereof such as rayon, pulp, and cotton; and polyacrylonitrile. One of these materials may be used alone, or two or more thereof may be used in combination. As the support layer according to the present invention, a fabric made of fibers of any of the above-described materials is preferably used. It is more preferable to use, as the fabric, a woven fabric or a nonwoven fabric obtained by processing the fibers by knitting (weaving), entanglement, thermal melt-bonding, compression bonding, binder bonding, or the like.

For example, a nonwoven fabric made of polyester fibers is preferably used as the support layer. A mass per unit area of the nonwoven fabric is more preferably 50 to 200 g/m$^2$. If the mass per unit area of the nonwoven fabric is less than the lower limit, there tend to arise such a problem of strength that the patch is easily broken during peeling off and such a problem that components contained in the adhesive agent layer seep to the back surface of the support layer, causing deterioration in the appearance and in the sense of use of the patch. Meanwhile, if the mass per unit area exceeds the upper limit, the stretchability and flexibility of the support layer tend to be so insufficient that the patch easily peels off.

The patch of the present invention may further comprise a release liner layer for covering and protecting a surface of the adhesive agent layer until the use of the patch. A material of the release liner layer is not particularly limited, and examples thereof include polyethylene, polypropylene, ethylene-vinyl acetate copolymer, ethylene-vinyl alcohol copolymer, vinyl chloride, polyurethane, polyesters such as polyethylene terephthalate, polybutylene terephthalate, and polyethylene naphthalate; polyamides such as nylon; polyacrylonitrile; celluloses and derivatives thereof; and foil of metals such as aluminum. One of these materials may be used alone, or two or more thereof may be used in combination. The release liner layer may be a film made of any of the above-described materials, and the film may be a film in which a surface to be in contact with the adhesive agent layer is subjected in-advance to a silicone treatment, a fluororesin treatment, or the like to increase the releasability of the film. As the release liner layer, it is preferable to use a film made of polyethylene terephthalate or polypropylene. In addition, a thickness of the release liner layer is preferably 20 to 150 $\mu$m.

The patch of the present invention can be produced by employing, as appropriate, a known method for producing a patch, without any particular limitation. For example, when a poultice is produced as the patch of the present invention, the poultice can be produced by the following -method. Specifically, first, the diclofenac or the pharmaceutically acceptable salt thereof, the glycerin, the butylene glycol, the poly(ethylene glycol) monooleate, the'water, the water-soluble polymer, and, if necessary, the additives, and the like are kneaded in a usual manner to obtain an uniform adhesive agent layer composition. Subsequently, this adhesive agent layer composition is applied onto a surface or surfaces (generally on one of the surfaces) of the support layer to a predetermined thickness to form the adhesive agent layer. Subsequently, the release liner layer is laminated on a surface of the adhesive agent layer opposite to the support layer, and these laminated layers are cut into a predetermined shape. Thus, the patch of the present invention can be obtained. Alternatively, the patch of the present invention may be obtained as follows. Specifically, the adhesive agent layer composition is first applied onto one surface of the release liner layer to a predetermined thickness to form the adhesive agent layer. Then, the support layer is laminated on a surface of the adhesive agent layer opposite to the release liner layer, and these laminated layers are cut intro a predetermined shape.

[Examples]

Hereinafter, the present invention is described more specifically based on Examples and Comparative Examples. However, the present invention is not limited to Examples below. Note that a skin permeation test, an adhesive force test (rolling ball tack test), a shape retainability evaluation test, an adhesiveness evaluation test, an evaluation test of pain during peeling off, a stability evaluation test, and an evaluation test of coloring over time were conducted on patches obtained in Examples and Comparative Examples by the following methods.

(Skin Permeation Test)

First, the skin on the back of a hairless mouse was peeled off, and set to a Franz-type flow-through cell in which hot water of 37°C was circulated through an outer peripheral portion, while the dermis side of the skin was located on the receptor chamber side. Subsequently, a patch which was cut into a size of 4.5 cm$^2$ and from which the release liner layer was removed was laminated on the corneum side of the skin. A phosphate buffer solution (pH 7.4) was caused to flow at a flow rate of 5 mL/hr in the receptor chamber of the flow-through cell, and a sample liquid was collected every 4 hours from the receptor chamber for 12 hours from the start of the measurement. Each of the collected sample liquids were measured for the concentration of the drug (diclofenac) by high-performance liquid chromatography. From the measurement values, the skin permeation amount (F: $\mu$g/cm$^2$/hr) of the drug was determined for each hour by using the following formula:

$$F\ (\mu g/cm^2/hr) = [drug\ concentration\ (\mu g/ml)\ \times\ flow$$
$$volume\ (ml)]/patch\ area\ (cm^2)/time\ (hr).$$

By summing up the obtained skin permeation amounts of the drug in 12 hours, the cumulative skin permeation amount ($\mu$g/cm$^2$/12 hr) of the drug in 12 hours from the start of the measurement was found. In addition, by summing up the obtained permeation amounts of the drug in 24 hours, the cumulative skin permeation amount ($\mu$g/cm$^2$/24 hr) of the drug in 24 hours from the start of the measurement was found. It can be understood that a pharmaceutical preparation with a larger value of the cumulative skin permeation amount was better in skin permeability of the drug.

(Rolling Ball Tack Test)

Measurement was conducted by the Nichiban Rolling Ball method ("SECCHAKU BINRAN (Adhesion Handbook)" 14th edition, published by PORIMA KANKOUKAI (polymer publishing group), 1985). Specifically, a patch from which the release liner layer was removed was placed, with the adhesive agent layer upside, on a horizontal bottom portion of an inclined stage having a sine curve shape. Then, a No. 20 steel ball (diameter: 20/32 inches) was rolled on an inclined surface of the inclined stage, and the distance at which the ball stopped on the adhesive agent layer was measured and was employed as the ball stop distance. Note that it can be understood that a pharmaceutical preparation with a shorter ball stop distance was better in adhesiveness.

(Shape Retainability Evaluation Test)

A surface of an adhesive agent layer of a patch from which a release liner layer was removed was touched lightly with a finger, and the finger was detached, and an evaluation was made based on the following criteria:

A: When the finger was detached, the adhesive agent layer was not distorted, and kept the shape.
B: When the finger was detached, the adhesive agent layer was partially distorted and peeled off.

(Adhesiveness Evaluation Test)

A patch was attached to an elbow of each of 30 healthy adult males and females, and the elbow was bent and straightened 40 times. Then, evaluation was made based on the following criteria:

Score 1: The patch was peeled off in approximately 1/2 or more of the area of the patch.
Score 2: The patch was peeled off in approximately 1/3 or more and less than 1/2 of the area of the patch.

Score 3: The patch was peeled off in approximately 1/4 or more and less than 1/3 of the area of the patch.
Score 4 : The patch was peeled off only in an end portion or end portions.
Score 5: The patch was not peeled off in any portion.

Then, the average value of the scores was found and employed as an initial adhesiveness.
Moreover, a patch was attached to an elbow of each of 30 healthy adult males and females, and 12 hours later evaluation was made based on the following criteria:

Score 1: The patch fell off, and was not attached.
Score 2: The patch was peeled off in approximately 1/2 or more of the area of the patch.
Score 3: The patch was peeled off in approximately 1/3 or more and less' than 1/2 of the area of the patch.
Score 4: The patch was peeled off only in an end portion or end portions.
Score 5: The patch was not peeled off in any portion.

Then, the average value of the scores was found, and employed as a 12-hour adhesiveness.

(Evaluation Test of Pain during Peeling Off)

A patch was attached to an elbow of each of 30 healthy adult males and females, and the patch was peeled off 12 hours later, and evaluated based on the following criteria:

Score 1: Maximum pain (extremely strong pain was felt)
Score 2: Very painful (strong pain was felt)
Score 3: Painful (pain was felt)
Score 4: Slightly painful (weak pain was felt)
Score 5: No pain (no particular pain was felt)

Then, the average value of the scores was found, and employed as the evaluation of pain during peeling off.

(Stability Evaluation Test)

A patch was sealed in an aluminum package, and left to stand still in a constant temperature and humidity chamber at a temperature of 60°C and a humidity of 75%. After a month had elapsed, the patch was taken out, and the content (remaining mass) of the drug (diclofenac sodium) was determined by high-performance liquid chromatography. In addition, for a patch immediately after production, the content (initial mass) of the drug was determined by high-performance liquid chromatography in the same manner. Then, the amount of remaining diclofenac sodium (% by mass) was found by using the following formula: (remaining mass/initial mass) $\times 100$. Note that it can be understood that a pharmaceutical preparation with a large amount of remaining diclofenac sodium was excellent in stability.

(Evaluation Test of Coloring over Time)

First, patches were stored by being allowed to stand still at 50°C or 60°C for 15 days (0.5 months). Subsequently, by using a color and color difference meter (trade name: CR-200, manufactured by Minolta Co., Ltd.) calibrated using a standard white plate, the coordinate (L, a, b) in the L*a*b* colorimetric system was determined by applying a measuring probe to the release liner side of a patch, which was not stored. In addition, the coordinate (L', a', b') was determined for each of the patches stored under the temperature conditions in the same manner. Then, the color difference ($\Delta E$) between the patch before the storage and each of the patches after the storage was calculated by using the following formula:

$$\Delta E = ((L-L')^2 + (a-a')^2 + (b-b')^2)^{1/2}.$$

Note that, in general, if the value of $\Delta E$ exceeds 1.5, the color difference can be recognized by the naked eyes. For reference, a general relationship between the $\Delta E$ value and recognition by the naked eyes is shown below.

$$0 \leq \Delta E < 1.5$$

Slightly different

$$1.5 \leq \Delta E < 3.0$$

Different to an extent that the difference can be noticed by the naked eyes

$$3.0 \leq \Delta E < 6.0$$

Remarkably different

$$6.0 \leq \Delta E < 12.0$$

Extremely remarkably different

$$12 \leq \Delta E$$

Recognized as a different color system

(Example 1)

First, an adhesive agent layer (drug-containing layer) composition was obtained by weighing and mixing together 5.0 parts by mass of glycerin, 35.0 parts by mass of an aqueous D-sorbitol solution (70% by mass), 10.0 parts by mass of butylene glycol (1,3-butyleneglycol), 5.0 parts by mass of poly(ethylene glycol) monooleate (an average number of moles of oxyethylene groups added was 6), 1.0 parts by mass of diclofenac sodium, 0.5 parts by mass of menthol, 7.0 parts by mass of a water-soluble polymer 1 (mass of sodium polyacrylate:mass of carboxymethyl cellulose sodium salt=5:2), 0.5 parts by mass of a tackiness-improving agent, 6.0 parts by mass of an inorganic filler, 0.44 parts by mass of a cross-linking agent, 0.25 parts by mass of a pH-adjusting agent, 0.02 parts by mass of sodium sulfite, 0.02 parts by mass of dibutylhydroxytoluene, and 29.27 parts by mass of purified water. Subsequently, the obtained adhesive agent layer composition was spread on one surface of a nonwoven fabric (mass per unit area: 100 g/m$^2$) made of polyester fibers at 1000 g/m$^2$, and then the surface on which the adhesive agent layer composition was applied was covered with a polypropylene release liner layer. Then, patches (poultice) were obtained by cutting into a predetermined size (10 cm$\times$14 cm). Table 1 shows the results of the skin permeation test (12 hours) and the rolling ball tack test conducted on the obtained patches, as well as the constitution of the adhesive agent layer composition.

(Comparative Examples 1 to 17)

Patches (poultices) were obtained in the same manner as in Example 1, except that the constitution of the adhesive agent layer composition was changed to the constitutions shown in Tables 1 to 3. Tables 1 to 3 show the results of the skin permeation test (12 hours) and the rolling ball tack test conducted on the obtained patches, as well as the constitutions of the adhesive agent layer compositions. Note that compounds listed in Tables 1 to 3 are as follows.

POE(9) lauryl ether: polyoxyethylene lauryl ether (average number of moles of oxyethylene groups added: 9)
POE(2) oleyl ether: polyoxyethylene oleyl ether (average number of moles of oxyethylene groups added: 2)
POE(20) sorbitan monooleate: polyoxyethylene sorbitan monooleate (average number of moles of oxyethylene groups added: 20)
POE(7) oleyl ether: polyoxyethylene oleyl ether (average number of moles of oxyethylene groups added: 7)
POE(20) sorbitan trioleate: polyoxyethylene sorbitan trioleate (average number of moles of oxyethylene groups added: 20)
Polyethylene glycol (10) monolaurate: polyethylene glycol monolaurate (average number of moles of oxyethylene groups added: 10).

[Table 1]

| Constitution [parts by mass] | Example 1 | Comp. Ex. 1 | Comp. Ex. 2 | Comp. Ex. 3 | Comp. Ex. 4 | Comp. Ex. 5 | Comp. Ex. 6 |
|---|---|---|---|---|---|---|---|
| Glycerin | 5.0 | 5.0 | 15.0 | 5.0 | 5.0 | 5.0 | 5.0 |
| Aqueous D-sorbitol solution (70% by mass) | 35.0 | 35.0 | 35.0 | 35.0 | 35.0 | 35.0 | 35.0 |
| Propylene glycol | - | 10.0 | - | - | - | - | - |
| Butylene glycol | 10.0 | - | - | 10.0 | - | - | - |
| Dipropylene glycol | - | - | - | - | 10.0 | - | - |
| Polyethylene glycol 400 | - | - | - | - | - | 10.0 | - |
| Polyethylene glycol 200 | - | - | - | - | - | - | 10.0 |
| Poly(ethylene glycol) monooleate | 5.0 | - | - | - | - | - | - |
| POE(9) lauryl ether | - | 2.5 | 2.5 | 2.5 | 2.5 | 2.5 | 2.5 |
| Diisopropyl adipate | - | 2.5 | 2.5 | 2.5 | 2.5 | 2.5 | 2.5 |
| Diclofenac sodium | 1.0 | 1.0 | 1.0 | 1.0 | 1.0 | 1.0 | 1.0 |
| Menthol | 0.5 | 0.5 | 0.5 | 0.5 | 0.5 | 0.5 | 0.5 |
| Water-soluble polymer 1 | 7.0 | 7.0 | 7.0 | 7.0 | 7.0 | 7.0 | 7.0 |
| Tackiness-improving agent | 0.5 | 0.5 | 0.5 | 0.5 | 0.5 | 0.5 | 0.5 |
| Inorganic filler | 6.0 | 6.0 | 6.0 | 6.0 | 6.0 | 6.0 | 6.0 |
| Cross-linking agent | 0.44 | 0.44 | 0.44 | 0.44 | 0.44 | 0.44 | 0.44 |
| pH-adjusting agent | 0.25 | 0.25 | 0.25 | 0.25 | 0.25 | 0.25 | 0.25 |
| Sodium sulfite | 0.02 | 0.02 | 0.02 | 0.02 | 0.02 | 0.02 | 0.02 |
| Dibutylhydroxytoluene | 0.02 | 0.02 | 0.02 | 0.02 | 0.02 | 0.02 | 0.02 |
| Purified water | 29.27 | 29.27 | 29.27 | 29.27 | 29.27 | 29.27 | 29.27 |
| Total | 100 | 100 | 100 | 100 | 100 | 100 | 100 |
| Cumulative skin permeation amount ($\mu$g/cm$^2$/12 hr) | 18.9 | 7.2 | 3.3 | 8.7 | 6.4 | 2.4 | 3.2 |
| Ball stop distance (mm) | 32.0 | 39.7 | 36.4 | 42.5 | 49.2 | 54.2 | 43.5 |

[Table 2]

| Constitution [parts by mass] | Comp. Ex. 7 | Comp. Ex. 8 | Comp. Ex. 9 | Comp. Ex. 10 | Comp. Ex. 11 | Comp. Ex. 12 |
|---|---|---|---|---|---|---|
| Glycerin | 15.0 | 15.0 | 15.0 | 15.0 | 15.0 | 15.0 |
| Aqueous D-sorbitol solution (70% by mass) | 35.0 | 35.0 | 35.0 | 35.0 | 35.0 | 35.0 |
| POE(9) lauryl ether | 2.5 | 2.5 | 5.0 | 2.5 | 2.5 | 2.5 |
| Poly(ethylene glycol) monooleate | 2.5 | 5.0 | - | - | - | - |
| Diisopropyl adipate | - | - | - | - | - | - |

(continued)

| Constitution [parts by mass] | Comp. Ex. 7 | Comp. Ex. 8 | Comp. Ex. 9 | Comp. Ex. 10 | Comp. Ex. 11 | Comp. Ex. 12 |
|---|---|---|---|---|---|---|
| N-methyl-2-pyrrolidone | - | - | - | 2.5 | - | - |
| Polyethylene glycol 400 | - | - | - | - | 2.5 | - |
| POE(2) oleyl ether | - | - | - | - | - | 2.5 |
| Diclofenac sodium | 1.0 | 1.0 | 1.0 | 1.0 | 1.0 | 1.0 |
| Menthol | 0.5 | 0.5 | 0.5 | 0.5 | 0.5 | 0.5 |
| water-soluble polymer 1 | 7.0 | 7.0 | 7.0 | 7.0 | 7.0 | 7.0 |
| Tackiness-improving agent | 0.5 | 0.5 | 0.5 | 0.5 | 0.5 | 0.5 |
| Inorganic filler | 6.0 | 6.0 | 6.0 | 6.0 | 6.0 | 6.0 |
| Cross-linking agent | 0.44 | 0.44 | 0.44 | 0.44 | 0.44 | 0.44 |
| pH-adjusting agent | 0.25 | 0.25 | 0.25 | 0.25 | 0.25 | 0.25 |
| Sodium sulfite | 0.02 | 0.02 | 0.02 | 0.02 | 0.02 | 0.02 |
| Dibutylhydroxytoluene | 0.02 | 0.02 | 0.02 | 0.02 | 0.02 | 0.02 |
| Purified water | 29.27 | 26.77 | 29.27 | 29.27 | 29.27 | 29.27 |
| Total | 100 | 100 | 100 | 100 | 100 | 100 |
| Cumulative skin permeation amount ($\mu$g/cm$^2$/12 hr) | 5.3 | 6.8 | 2.4 | 4.1 | 3.5 | 3.4 |
| Ball stop distance (mm) | 24.3 | - | 24.3 | 32.6 | 32.6 | 28.5 |

[Table 3]

| Constitution [parts by mass] | Comp. Ex. 13 | Comp. Ex. 14 | Comp. Ex. 15 | Comp. Ex. 16 | Comp. Ex. 17 |
|---|---|---|---|---|---|
| Glycerin | 15.0 | 15.0 | 15.0 | 15.0 | 15.0 |
| Aqueous D-sorbitol solution (70% by mass) | 35.0 | 35.0 | 35.0 | 35.0 | 35.0 |
| POE(9) lauryl ether | 2.5 | 2.5 | 2.5 | 2.5 | 2.5 |
| Poly(ethylene glycol) monooleate | - | - | - | - | - |
| Diisopropyl adipate | - | - | - | - | - |
| 3-L-Menthoxypropane-1,2-diol | 2.5 | - | - | - | - |
| POE(20) sorbitan monooleate | - | 2.5 | - | - | - |
| POE(7) oleyl ether | - | - | 2.5 | - | - |
| POE(20) sorbitan trioleate | - | - | - | 2.5 | - |
| Polyethylene glycol(10) monolaurate | - | - | - | - | 2.5 |
| Propylene glycol | - | - | - | - | - |
| Butylene glycol | - | - | - | - | - |
| Diclofenac sodium | 1.0 | 1.0 | 1.0 | 1.0 | 1.0 |
| Menthol | 0.5 | 0.5 | 0.5 | 0.5 | 0.5 |
| Water-soluble polymer 1 | 7.0 | 7.0 | 7.0 | 7.0 | 7.0 |
| Tackiness-improving agent | 0.5 | 0.5 | 0.5 | 0.5 | 0.5 |

(continued)

| Constitution [parts by mass] | Comp. Ex. 13 | Comp. Ex. 14 | Comp. Ex. 15 | Comp. Ex. 16 | Comp. Ex. 17 |
|---|---|---|---|---|---|
| Inorganic filler | 6.0 | 6.0 | 6.0 | 6.0 | 6.0 |
| Cross-linking agent | 0.44 | 0.44 | 0.44 | 0.44 | 0.44 |
| pH-adjusting agent | 0.25 | 0.25 | 0.25 | 0.25 | 0.25 |
| Sodium sulfite | 0.02 | 0.02 | 0.02 | 0.02 | 0.02 |
| Dibutylhydroxytoluene | 0.02 | 0.02 | 0.02 | 0.02 | 0.02 |
| Purified water | 29.27 | 29.27 | 29.27 | 29.27 | 29.27 |
| Total | 100 | 100 | 100 | 100 | 100 |
| Cumulative skin permeation amount ($\mu g/cm^2/12$ hr) | 2.0 | 2.0 | 2.4 | 2.0 | 2.6 |
| Ball stop distance (mm) | 29.8 | 27.2 | 27.5 | 25.8 | 25.3 |

As is apparent from the results shown in Table 1, it was found that the patch of the present invention achieved a remarkably good skin permeability of diclofenac, although the patch of the present invention contained glycerin, and did not contain propylene glycol or the like causing skin irritation. Meanwhile, it was found that when other humectants (Comparative Example 2 to 6) were blended instead of propylene glycol (Comparative Example 1), the cumulative skin permeation amount of diclofenac tended to decrease, and especially that when simply glycerin was used (Comparative Example 2) instead of propylene glycol, the cumulative skin permeation amount of diclofenac greatly decreased. In addition, it was found that when simply butylene glycol was used (Comparative Example 3) instead of propylene glycol, the cumulative skin permeation amount of diclofenac was not improved. Note that the patch obtained in Comparative Example 3 had strong odor of diisopropyl adipate, which made it difficult to use the patch as a pharmaceutical preparation. Moreover, as is apparent from the results shown in Tables 2 and 3, it was found that when the butylene glycol according to the present invention was not contained, but simply solubilizers were used (Comparative Example 7 to 17), the cumulative skin permeation amount of diclofenac was not improved, even when poly(ethylene glycol) monooleate was used (Comparative Example 7 and 8). Accordingly, it has been found that the effect of improving the skin permeability of diclofenac achieved by the patch of the present invention is a synergistic effect.

(Examples 2 to 6)

Patches (poultices) were obtained in the same manner as in Example 1, except that the constitution of the adhesive agent layer composition was changed to the constitutions shown in Table 4. Table 4 shows the results of the skin permeation test (12 hours), the rolling ball tack test, and the evaluation test of coloring over time conducted on the obtained patches, as well as the constitutions of the adhesive agent layer compositions. Note that the water-soluble polymer 2 listed in Table 4 contained sodium polyacrylate, carboxymethyl cellulose sodium salt, gelatin, and hydroxypropyl cellulose at a mass ratio of 5:1.25:2:3 in this order.

[Table 4]

| Constitution [parts by mass] | Example 2 | Example 3 | Example 4 | Example 5 | Example 6 |
|---|---|---|---|---|---|
| Glycerin | 5.0 | 5.0 | 5.0 | 5.0 | 5.0 |
| Aqueous D-sorbitol solution (70% by mass) | 35.0 | 35.0 | 35.0 | 35.0 | 35.0 |
| Butylene glycol | 10.0 | 10.0 | 10.0 | 10.0 | 10.0 |
| Poly(ethylene glycol) monooleate | 4.0 | 4.0 | 4.0 | 4.0 | 4.0 |
| Diclofenac sodium | 1.0 | 1.0 | 1.0 | 1.0 | 1.0 |
| Menthol | 0.5 | 0.5 | 0.5 | 0.5 | 0.5 |
| Water-soluble polymer 2 | 11.25 | 11.25 | 11.25 | 11.25 | 11.25 |
| Tackiness-improving agent | 0.5 | 0.5 | 0.5 | 0.5 | 0.5 |

(continued)

| Constitution [parts by mass] | Example 2 | Example 3 | Example 4 | Example 5 | Example 6 |
|---|---|---|---|---|---|
| Inorganic filler | 6.0 | 6.0 | 6.0 | 6.0 | 6.0 |
| Cross-linking agent | 0.33 | 0.33 | 0.33 | 0.33 | 0.33 |
| pH-adjusting agent | 0.45 | 0.45 | 0.45 | 0.45 | 0.45 |
| Sodium sulfite | 0.20 | 0.02 | 0.02 | 0.02 | 0.02 |
| Sodium hydrogen sulfite | - | 0.30 | - | - | - |
| Sodium pyrosulfite | - | - | 0.15 | - | - |
| Sodium citrate | - | - | - | 0.70 | - |
| Dibutylhydroxytoluene | 0.02 | 0.02 | 0.02 | 0.02 | 0.02 |
| Purified water | 25.75 | 25.63 | 25.78 | 25.23 | 25.93 |
| Total | 100 | 100 | 100 | 100 | 100 |
| Cumulative skin permeation amount ($\mu$g/cm$^2$/12 hr) | 10.6 | - | - | - | - |
| Ball stop distance (mm) | 26.0 | 30.0 | - | - | - |
| Color difference ($\Delta$E, 50°C, 0.5 months) | 0.32 | 0.68 | 0.50 | 1.68 | 2.16 |
| Color difference ($\Delta$E, 60°C, 0.5 months) | 1.14 | 0.80 | 1.38 | 6.28 | 6.64 |

As is apparent from the results shown in Table 4, it was found that the change in color of the adhesive agent layer was suppressed especially in the patch (Example 2) containing a large amount of sodium sulfite and the patches (Examples 3 and 4) containing sodium sulfite in combination with sodium pyrosulfite or sodium hydrogen sulfite.

(Examples 7 to 19 and Comparative Examples 18 and 19)

Patches (poultices) were obtained in the same manner as in Example 1, except that the constitution of the adhesive agent layer composition is changed to the constitutions shown in Tables 5 and 6. Table 5 shows the results of the skin permeation test (12 hours), the rolling ball tack test, and the shape retainability evaluation test conducted on the patches obtained in Examples 7 to 9 and Comparative Examples 18 and 19, as well as the constitutions of the adhesive agent layer compositions. In addition, Table 5 also shows the results of Example 2. Moreover, Table 6 shows the results of the rolling ball tack test conducted on the patches obtained in Examples 10 to 19, as well as the constitutions of the adhesive agent layer compositions.

[Table 5]

| Constitution [parts by mass] | Comp. Ex. 18 | Example 7 | Example 8 | Example 2 | Example 9 | Comp. Ex. 19 |
|---|---|---|---|---|---|---|
| Glycerin | 5.0 | 5.0 | 5.0 | 5.0 | 5.0 | 5.0 |
| Aqueous D-sorbitol solution (70% by mass) | 35.0 | 35.0 | 35.0 | 35.0 | 35.0 | 35.0 |
| Butylene glycol(A) | 2.0 | 4.0 | 5.0 | 10.0 | 16.0 | 20.0 |
| Poly(ethylene glycol) monooleate(B) | 4.0 | 4.0 | 4.0 | 4.0 | 4.0 | 4.0 |
| Diclofenac sodium | 1.0 | 1.0 | 1.0 | 1.0 | 1.0 | 1.0 |
| Menthol | 0.5 | 0.5 | 0.5 | 0.5 | 0.5 | 0.5 |
| Water-soluble polymer 2 | 11.25 | 11.25 | 11.25 | 11.25 | 11.25 | 11.25 |
| Tackiness-improving agent | 0.5 | 0.5 | 0.5 | 0.5 | 0.5 | 0.5 |
| Inorganic filler | 6.0 | 6.0 | 6.0 | 6.0 | 6.0 | 6.0 |
| Cross-linking agent | 0.33 | 0.33 | 0.33 | 0.33 | 0.33 | 0.33 |

(continued)

| Constitution [parts by mass] | Comp. Ex. 18 | Example 7 | Example 8 | Example 2 | Example 9 | Comp. Ex. 19 |
|---|---|---|---|---|---|---|
| pH-adjusting agent | 0.45 | 0.45 | 0.45 | 0.45 | 0.45 | 0.45 |
| Sodium sulfite | 0.20 | 0.20 | 0.20 | 0.20 | 0.20 | 0.20 |
| Dibutylhydroxytoluene | 0.02 | 0.02 | 0.02 | 0.02 | 0.02 | 0.02 |
| Purified water | 33.75 | 31.75 | 30.75 | 25.75 | 19.75 | 15.75 |
| Total | 100 | 100 | 100 | 100 | 100 | 100 |
| Mass ratio between A and B (A:B) | 0.5:1 | 1:1 | 1.25:1 | 2.5:1 | 4:1 | 5:1 |
| Cumulative skin permeation amount ($\mu$g/cm$^2$/12 hr) | 6.6 | 8.6 | 10.8 | 10.6 | 16.3 | 18.9 |
| Ball stop distance (mm) | 30.7 | 27.3 | 22.3 | 26.0 | - | - |
| Shape retainability evaluation | A | A | A | A | A | B |

[Table 6]

| Constitution [parts by mass] | Ex. 10 | Ex. 11 | Ex. 12 | Ex. 13 | Ex. 14 | Ex. 15 | Ex. 16 | Ex. 17 | Ex. 18 | Ex. 19 |
|---|---|---|---|---|---|---|---|---|---|---|
| Glycerin | 30.0 | 30.0 | 30.0 | 28.5 | 33.7 | 28.5 | 30.0 | 30.0 | 30.0 | 27.5 |
| Butylene glycol | 5.0 | 5.0 | 5.0 | 5.0 | 7.5 | 5.0 | 5.0 | 5.0 | 5.0 | 5.0 |
| Polyethylene glycol monooleate | 4.0 | 4.0 | 4.0 | 4.0 | 4.0 | 4.0 | 4.0 | 4.0 | 4.0 | 4.0 |
| Diclofenac sodium | 1.0 | 1.0 | 1.0 | 1.0 | 1.0 | 1.0 | 1.0 | 1.0 | 1.0 | 1.0 |
| Menthol | 0.5 | 0.5 | 0.5 | 0.5 | 0.5 | 0.5 | 5.0 | 0.5 | 0.5 | 0.5 |
| Sodium polyacrylate | 5.0 | 5.0 | 5.0 | 5.0 | 5.0 | 5.0 | 5.0 | 5.0 | 5.0 | 5.0 |
| Carboxymethyl cellulose sodium salt | 1.25 | 1.25 | 1.25 | 1.25 | 1.25 | 1.25 | 1.25 | 1.25 | 1.25 | 1.25 |
| Gelatin | 2.00 | 2.00 | 2.00 | 2.00 | 2.00 | 2.00 | 1.60 | 1.00 | 0.68 | 1.50 |
| Hydroxypropyl cellulose | 3.0 | 3.0 | 3.0 | 3.0 | 3.0 | 3.0 | 3.0 | 3.0 | 3.0 | 3.0 |
| Polyvinyl alcohol | 2.0 | 2.0 | 3.0 | 3.0 | 3.0 | 2.0 | 2.0 | 2.0 | 2.0 | 2.0 |
| Tackiness-improving agent | 0.5 | 0.5 | 0.5 | 0.5 | 0.5 | 0.5 | 0.5 | 0.5 | 0.5 | 0.5 |
| Inorganic filler | 6.0 | 6.0 | 6.0 | 6.0 | 6.0 | 6.0 | 6.0 | 6.0 | 6.0 | 6.0 |
| Cross-linking agent | 0.33 | 0.38 | 0.33 | 0.33 | 0.38 | 0.38 | 0.41 | 0.41 | 0.41 | 0.37 |
| pH-adjusting agent | 0.22 | 0.20 | 0.22 | 0.25 | 0.25 | 0.05 | 0.17 | 0.17 | 0.17 | 0.20 |
| Sodium sulfite | 0.20 | 0.20 | 0.20 | 1.00 | 1.00 | 1.00 | 0.20 | 0.20 | 0.20 | 0.20 |
| Dibutylhydroxytoluene | 0.02 | 0.02 | 0.02 | 0.02 | 0.02 | 0.02 | 0.02 | 0.02 | 0.02 | 0.02 |
| Purified water | 38.98 | 38.95 | 37.98 | 38.65 | 30.90 | 39.80 | 39.36 | 39.96 | 40.28 | 41.97 |
| Total | 100 | 100 | 100 | 100 | 100 | 100 | 100 | 100 | 100 | 100 |
| Ball stop distance (mm) | 35.7 | - | 30.3 | - | - | - | 33.7 | 29.7 | 25.0 | 36.7 |

**EP 2 789 335 B1**

As is apparent from the results shown in Table 5, it was found that the skin permeability of diclofenac was not sufficiently improved by the patch (Comparative Example 18) having a low ratio of the mass of butylene glycol to the mass of poly(ethylene glycol) monooleate. In addition, the patch (Comparative Example 19) having a high ratio of the mass of butylene glycol to the mass of poly(ethylene glycol) monooleate was poor in shape retainability of the adhesive agent layer, and was difficult to use as a patch.

Moreover, Table 7 shows the results of the skin permeation test (24 hours), the adhesiveness evaluation test, the evaluation test of pain during peeling off, and the stability evaluation test conducted on the patches obtained in Comparative Example 1, and Examples 2 to 3, 8, and 10 to 15.

[Table 7]

| | Example 2 | Example 3 | Example 8 | Example 10 | Example 11 |
|---|---|---|---|---|---|
| Cumulative skin permeation amount ($\mu$ g/cm$^2$/24 hr) | - | - | 32.1 | 42.0 | - |
| Initial adhesiveness | 4.4 | 4.3 | - | 4.4 | 4.0 |
| 12-hour adhesiveness | 4.2 | 4.1 | - | 3.9 | 3.9 |
| Evaluation of pain during peeling off | 3.7 | 3.7 | - | 4.1 | 3.8 |
| Amount of remaining diclofenac sodium (% by mass) | - | - | - | 95.4 | 96.3 |
| | Example 12 | Example 13 | Example 14 | Example 15 | Comp. Ex. 1 |
| Cumulative skin permeation amount ($\mu$ g/cm$^2$/24 hr) | - | - | - | - | 20.5 |
| Initial adhesiveness | 4.2 | - | - | - | 3.5 |
| 12-hour adhesiveness | 3.8 | - | - | - | 3.3 |
| Evaluation of pain during peeling off | 4.1 | - | - | - | 3.0 |
| Amount of remaining diclofenac sodium (% by mass) | 95.0 | 95.3 | 93.5 | 92.0 | 95.6 |

As is apparent from the results of the evaluation test of pain during peeling off shown in Table 7, it was found that the pain during peeling off of the patch of the present invention was sufficiently reduced. In addition, it was shown by the skin permeation test (24 hours) and the adhesiveness test that the patches of the present invention had excellent skin permeability and excellent adhesiveness. Moreover, it was found that the patches of the present invention were excellent in stability, and decomposition of the drug was sufficiently suppressed.

[Industrial Applicability]

As described above, according to the present invention, it is possible to provide a patch which gives reduced pain during peeling off and less irritation to the skin, and which has an excellent skin permeability of diclofenac and an excellent adhesiveness.

**Claims**

1. A patch comprising a support layer and an adhesive agent layer, wherein
   the adhesive agent layer contains diclofenac or a pharmaceutically acceptable salt thereof, glycerin, butyleneglycol,and poly(ethyleneglycol) monooleate, and
   a mass ratio between the butylene glycol and the poly(ethylene glycol) monooleate (mass of butylene glycol:mass of poly(ethylene glycol) monooleate) is 1:1 to 4:1.

2. The patch according to claim 1, wherein
   a content of the butylene glycol is 4 to 18% by mass relative to a total mass of the adhesive agent layer.

3. The patch according to claim 1 or 2, wherein

a content of the poly(ethylene glycol) monooleate is 2.5 to 7.5% by mass relative toa total mass of the adhesive agent layer.

**4.** The patch according to any one of claims 1 to 3, wherein the adhesive agent layer further contains sodium sulfite.

**5.** The patch according to any one of claims 1 to 4, wherein an average number of moles of oxyethylene groups added in the poly(ethylene glycol) monooleate is 2 to 10.

**6.** The patch according to any one of claims 1 to 5, wherein the butylene glycol is 1,3-butylene glycol.

**Patentansprüche**

**1.** Pflaster, umfassend eine Trägerschicht und eine Klebemittelschicht, wobei die Klebemittelschicht Diclofenac oder ein pharmazeutisch verträgliches Salz davon, Glycerin, Butylenglykol und Poly(ethylenglykol)monooleat enthält, und ein Massenverhältnis zwischen dem Butylenglykol und dem Poly(ethylenglykol)monooleat (Masse von Butylenglykol : Masse von Poly(ethylenglykol)monooleat) 1:1 bis 4:1 beträgt.

**2.** Pflaster nach Anspruch 1, wobei ein Gehalt des Butylenglykols 4 bis 18 Massen-% beträgt, bezogen auf die Gesamtmasse der Klebemittelschicht.

**3.** Pflaster nach Anspruch 1 oder 2, wobei ein Gehalt des Poly(ethylenglykol)monooleats 2,5 bis 7,5 Massen-% beträgt, bezogen auf eine Gesamtmasse der Klebemittelschicht.

**4.** Pflaster nach einem der Ansprüche 1 bis 3, wobei die Klebemittelschicht ferner Sodiumsulfit enthält.

**5.** Pflaster nach einem der Ansprüche 1 bis 4, wobei die durchschnittliche Anzahl von Mol an Oxyethylengruppen, zugegeben zu dem Poly(ethylenglykol)monooleat, 2 bis 10 beträgt.

**6.** Pflaster nach einem der Ansprüche 1 bis 5, wobei das Butylenglykol 1,3-Butylenglykol ist.

**Revendications**

**1.** Timbre comprenant une couche formant support et une couche d'agent adhésif, dans lequel la couche d'agent adhésif contient du diclofénac ou un sel pharmaceutiquement acceptable de celui-ci, de la glycérine, du butylène glycol, et du poly(monooléate d'éthylène glycol), et un rapport en masse entre le butylène glycol et le poly(monooléate d'éthylène glycol) (masse du butylène glycol:masse du poly(monooléate d'éthylène glycol)) est de 1:1 à 4:1.

**2.** Timbre selon la revendication 1, dans lequel une teneur du butylène glycol est de 4 à 18 % en masse par rapport à une masse totale de la couche d'agent adhésif.

**3.** Timbre selon la revendication 1 ou 2, dans lequel une teneur du poly(monooléate d'éthylène glycol) est de 2,5 à 7,5 % en masse par rapport à une masse totale de la couche d'agent adhésif.

**4.** Timbre selon l'une quelconque des revendications 1 à 3, dans lequel la couche d'agent adhésif contient en outre du sulfite de sodium.

**5.** Timbre selon l'une quelconque des revendications 1 à 4, dans lequel un nombre moyen de moles des groupes oxyéthylène ajoutés au poly(monooléate d'éthylène glycol) est de 2 à 10.

**6.** Timbre selon l'une quelconque des revendications 1 à 5, dans lequel le butylène glycol est le 1,3-butylène glycol.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP SHO63091318 B
- JP HEI656660 B
- JP HEI10182450 B
- JP 2002193793 A
- JP SHO61060608 B
- JP HEI3109321 B

**Non-patent literature cited in the description**

- SECCHAKU BINRAN (Adhesion Handbook). PORI-MA KANKOUKAI, 1985